# EUROPÄISCHE PATENTSCHRIFT

(11) **EP 1 458 295 B1**
(45) Veröffentlichungstag und Bekanntmachung des Hinweises auf die Patenterteilung: **21.03.2007**
(21) Anmeldenummer: 02785357.1
(22) Anmeldetag: 02.11.2002
(51) Int. Cl.: A61B 17/04

(54) **NAHT-SET FÜR MEDIZINISCHE ZWECKE**
SUTURING SET FOR MEDICAL USE
ENSEMBLE DE SUTURE DESTINE A DES APPLICATIONS MEDICALES

(30) Priorität: 27.11.2001 DE 10158142
(43) Veröffentlichungstag der Anmeldung: 22.09.2004
(73) Patentinhaber: Karl Storz GmbH & Co. KG, 78532 Tuttlingen (DE)
(72) Erfinder: SAUER, Michael, 78532 Tuttlingen (DE); OBERLÄNDER, Martin, 78532 Tuttlingen (DE)
(74) Vertreter: Hofmeister, Frank
(86) Internationale Anmeldenummer: PCT/EP2002/012236
(87) Internationale Veröffentlichungsnummer: WO 2003/045254

(56) Entgegenhaltungen:
- FR-A- 952 119
- FR-A- 1 069 680
- US-A- 2 808 055
- US-A- 3 013 559
- US-A- 5 928 252

## Beschreibung

Die Erfindung betrifft ein Naht-Set für medizinische Zwecke, mit einem Griffteil, einer an dem Griffteil festlegbaren, zumindest teilweise als Hohlnadel ausgebildeten Nadel sowie einer in dem Griffteil angeordneten, zwischen zwei Endlagen verschiebbaren Transportvorrichtung zum Zuführen von biegeschlaffem Nahtmaterial zur Nadel, wobei die Transportvorrichtung eine Einspannvorrichtung aufweist, über die das eingeführte Nahtmaterial in einer Endlage der Transportvorrichtung klemmend ergreifbar und in der anderen Endlage der Transportvorrichtung wieder freigebbar ist.

Ein gattungsgemäßes Naht-Set ist beispielsweise aus der FR 1 069 680 A bekannt. Bei dem aus dieser Druckschrift bekannten chirurgischen Naht-Set besteht die Transportvorrichtung aus einem in einer Führung verschiebbar gelagerten Schieber, der aus zwei Platten besteht, die über Federn voneinander beabstandet sind. Zwischen den beiden Platten des Schiebers ist ein Rohr zur Durchführung des Nahtmaterials gelagert. Zum Verschieben des Schiebers sowie zum Fixieren des Nahtmaterials innerhalb des Rohres weist die obere Platte einen Druckknopf auf, über den diese Platte gegen die Kraft der Feder in Richtung der unteren Platte verlagerbar ist. Bei dieser Betätigung des Druckknopfes tritt das konische spitz zulaufende Ende des Druckknopfes durch eine Öffnung in das Rohr ein und fixiert das in dem Rohr befindliche Nahtmaterial. Bei gedrücktem Druckknopf kann nun der Schieber mitsamt dem über den konischen Fortsatz des Druckknopfes ergriffenen Rohr und dem Nahtmaterial verschoben werden.

Diese bekannte Vorrichtung ermöglicht zwar einen überwiegend materialschonenden Transport des Nahtmaterials, jedoch weist dieses Naht-Set die Nachteile auf, dass einerseits der Druckknopf während der gesamten Zeit, während der der Knopf verschoben wird gedrückt gehalten werden muß, um das Nahtmaterial mitzunehmen und andererseits die Gefahr besteht, dass beim Zurückschieben des Schiebers der Druckknopf gedrückt wird und somit das Nahtmaterial wieder ergriffen und aus der Nadel herausgezogen wird.

Ein weiteres Naht-Set ist aus der US-Patentschrift 2 808 055 bekannt. Bei diesem bekannten Naht-Set besteht die Transportvorrichtung aus einem manuell in einer Führung des Griffteils verschiebbaren Gleitschuh mit einem röhrenförmigen Fortsatz. Der Gleitschuh sowie der Fortsatz sind mit einer Durchgangsbohrung zur Aufnahme des Nahtmaterials versehen. Beim Verschieben des Gleitschuhs wird der Fortsatz des Gleitschuhs in die Hohlnadel hineingeschoben bzw. wieder und aus dieser herausgezogen.

Das Zuführen des Nahtmaterials zur Nadel erfolgt bei dieser bekannten Anordnung dadurch, daß das Nahtmaterial ausgehend von einer im Griffteil angeordneten Garnspule zunächst aus dem Griffteil herausgeführt wird, um es dann über eine Fadenaufnahme in die Durchgangsbohrung des Gleitschuhs der Transportvorrichtung einzuführen. Beim Vorschieben des Gleitschuhs hin zur Nadel muß der Bediener des Naht-Sets das Nahtmaterial mit einem Finger an der Fadenaufnahme abknickend festklemmen, um sicherzustellen, daß das Nahtmaterial beim Vorschieben des Gleitschuhs mitgenommen wird. Das solchermaßen über den Gleitschuh und den röhrenförmigen Fortsatz in die Hohlnadel transportierte Nahtmaterial verklemmt sich dann durch Reibschluß derart im distalen engeren Teil der Hohlnadel, daß der Gleitschuh wieder zurückgeschoben werden kann, ohne das Nahtmaterial wieder aus der Hohlnadel herauszuziehen.

Nachteilig bei diesem bekannten Naht-Set ist, daß der Transport des Nahtmaterials hin zur Nadel nur dadurch ermöglicht wird, daß der Bediener des Sets das Nahtmaterial klemmend an der Fadenaufnahme halten muß. Hierdurch wird einerseits der Bediener des Sets bei der Handhabung des Naht-Sets eingeschränkt und besteht andererseits die Gefahr, daß das Nahtmaterial bei der knickenden Klemmung beschädigt wird. Darüber hinaus ist ein Wechsel des Nahtmaterials bei dieser Anordnung sehr zeit- und montageaufwendig, da das Griffteil zum Wechseln der Garnspule zerlegt werden muß.

Davon ausgehend liegt der Erfindung die **Aufgabe** zugrunde, ein Naht-Set der eingangs genannten Art derart zu verbessern, daß der Transport des Nahtmaterials zur Nadel einfach, sicher und materialschonend erfolgt.

Die **Lösung** dieser Aufgabenstellung ist erfindungsgemäß dadurch gekennzeichnet, daß die Transportvorrichtung derart ausgebildet ist, daß die Einspannvorrichtung eingeführtes Nahtmaterial beim Erreichen einer Endlage der Transportvorrichtung durch Verschieben der Transportvorrichtung klemmend ergreift und, daß das ergriffene Nahtmaterial erst beim Erreichen der anderen Endlage der Transportvorrichtung wieder freigebbar ist.

Aufgrund der erfindungsgemäßen Ausstattung der Transportvorrichtung mit einer Einspannvorrichtung für das Nahtmaterial ist es möglich, nach einem anfänglichen manuellen Einführen des Nahtmaterials den weiteren Transport des Nahtmaterials hin zur Nadel ohne einen weiteren manuellen Angriff am Nahtmaterial vorzunehmen.

Erfindungsgemäß ist ferner vorgesehen, daß die Einspannvorrichtung innerhalb eines manuell zwischen den beiden Endlagen der Transportvorrichtung verstellbaren Schiebers angeordnet ist, wobei die Einspannvorrichtung aus mindestens einem das Nahtmaterial umgebenden, radial elastisch verformbaren Zangenteil und mindestens einem relativ zu dem Zangenteil und auf das Zangenteil aufschiebbaren Klemmelement besteht. Sobald das mindestens eine Klemmelement auf das mindestens eine Zangenteil aufgeschoben wird, wird das Zangenteil derart radial verformt, daß das in dem Zangenteil geführt Nahtmaterial klemmend von dem Zangenteil ergriffen wird.

Das Verschieben des mindestens einen Klemmelements relativ zu dem mindestens einen Zangenteil erfolgt gemäß einer bevorzugten Ausführungsform der Erfindung derart, daß beim Anlaufen der Transportvorrichtung gegen die eine Endlage das Klemmelement auf das Zangenteil aufgeschoben wird und beim Anlaufen der Transportvorrichtung gegen die andere Endlage das Klemmelement wieder von dem Zangenteil herunter geschoben wird.

Gemäß einer praktischen Ausführungsform der Erfindung wird vorgeschlagen, daß die Einspannvorrichtung innerhalb eines manuell zwischen den beiden Endlagen der Transportvorrichtung verstellbaren Schiebers angeordnet ist.

Gemäß einer praktischen Ausgestaltungsform der Erfindung definieren im Griffteil angeordnete und am verschiebbaren Klemmelement angreifende Anschläge die Endlagen der Transportvorrichtung.

Das Aufschieben des mindestens einen Klemmelements auf das mindestens eine Zangenteil kann dadurch erleichtert werden, daß am Zangenteil und/oder am Klemmelement Anlaufschrägen ausgebildet sind.

Gemäß einer bevorzugten Ausführungsform der Erfindung ist die Nadel über ein Adapterstück am Griffteil festlegbar. Um beobachten zu können, ob die Transportvorrichtung das Nahtmaterial richtig eingezogen hat und knickfrei hin zur Nadel transportiert, ist in dem Adapterstück wenigstens eine einen Blick auf das Nahtmaterial freigebende Öffnung ausgebildet.

Weiterhin wird mit der Erfindung vorgeschlagen, daß im Übergangsbereich vom Adapterstück zur Nadel eine mit einem Schlitz zum Durchführen des Nahtmaterials versehene Dichtungskappe angeordnet ist. Diese Dichtungskappe, die zumindest im Bereich des Schlitzes aus einem elastischen Material, insbesondere einem Gummi- oder Kunststoff-Material, besteht, dient einerseits zum Verschließen des proximalen Endes der Nadel und andererseits mit dem Durchführungsschlitz für das Nahtmaterial als Klemmung und Halt für das eingeführte Nahtmaterial, da die Klemmung in dem Schlitz verhindert, daß das Nahtmaterial beim Zurückziehen der Transportvorrichtung, trotz geöffneter Zangenteile, durch Reibung im Rohrsystem der Transportvorrichtung wieder aus der Nadel zurückgezogen wird.

Um das Nahtmaterial innerhalb des Naht-Sets zu führen und zu stabilisieren, sind im Griffteil und im Adapterstück zumindest abschnittsweise Hohlrohre zur Aufnahme des Nahtmaterials angeordnet. Diese Hohlrohre verhindern ein Ausknicken und Beschädigen des Nahtmaterials.

Schließlich wird mit der Erfindung vorgeschlagen, daß zum Einführen des Nahtmaterials in das Griffteil und die Transportvorrichtung eine als radiale Bohrung ausgebildete Fadenaufnahme im Griffteil ausgebildet ist. Über diese Fadenaufnahme kann beliebiges Nahtmaterial von außen in die Transportvorrichtung eingeführt werden.

Weitere Merkmale und Vorteile der Erfindung ergeben sich anhand der nachfolgenden Beschreibung der zugehörigen Zeichnung, in der ein Ausführungsbeispiel eines erfindungsgemäßen Naht-Sets nur beispielhaft schematisch dargestellt ist. In der Zeichnung zeigt:
- Fig. 1: eine perspektivische Ansicht eines Griffteils eines erfindungsgemäßen Naht-Sets;
- Fig. 2a: einen ausschnittweisen Längsschnitt entlang der Schnittlinie IIa-IIa gemäß Fig. 1 durch das Griffteil mit aufgesetzter Nadel, die Transportvorrichtung in der hinteren Endlage darstellend;
- Fig. 2b: eine Schnittdarstellung gemäß Fig. 2b, jedoch die Transportvorrichtung in der vorderen Endlage darstellend;
- Fig. 3a: eine geschnittene perspektivische Detailansicht der Führung des Nahtmaterials, die Transportvorrichtung in der hinteren Endlage darstellend;
- Fig. 3b: eine perspektivische Detailansicht gemäß Fig. 3a, jedoch die Transportvorrichtung in der vorderen Endlage darstellend und
- Fig. 4: eine vergrößerte und vervollständigte Vorderansicht des Details IV gemäß Fig. 3a und 3b.

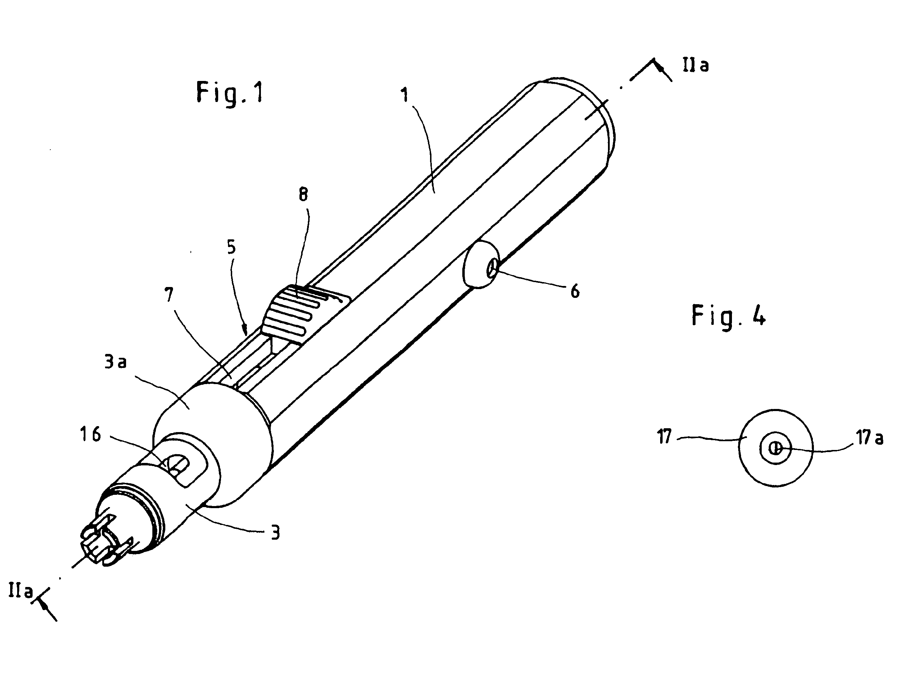

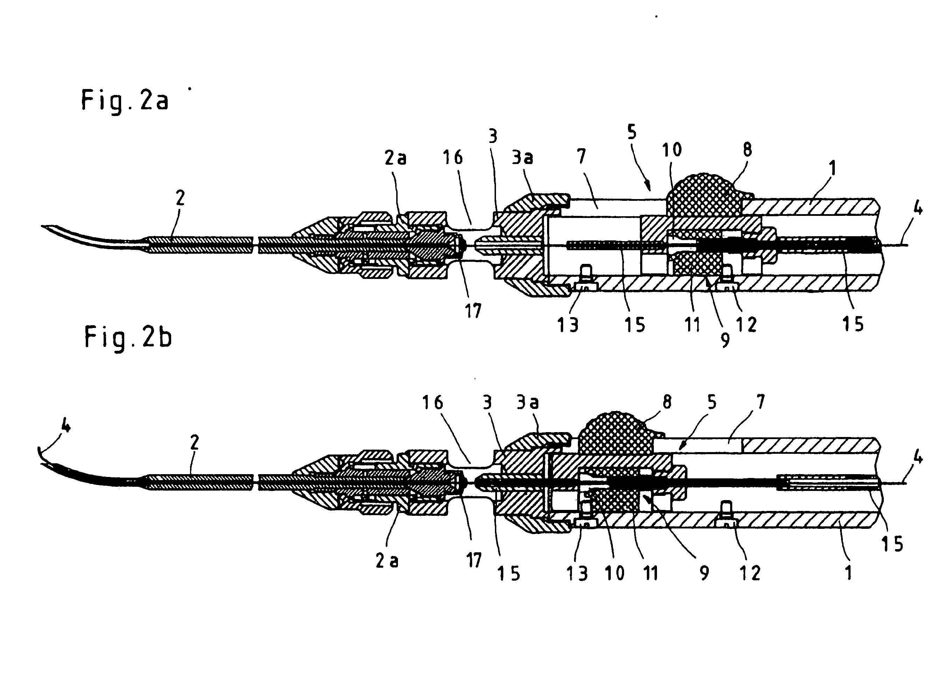

Fig. 1 zeigt in perspektivischer Ansicht ein Griffteil 1 eines Naht-Sets für medizinische Zwecke. Zur Vervollständigung des Naht-Sets ist am Griffteil 1 eine Nadel 2 festlegbar, die bei dem in den Abbildungen Fig. 2a und 2b dargestellten Ausführungsbeispiel als mit einer Durchgangsbohrung versehene Hohlnadel ausgebildet ist. Wie weiterhin aus Fig. 1 sowie Fig. 2a und 2b ersichtlich, sind bei dieser dargestellten Ausführungsform das Griffteil 1 und die Nadel 2 über ein zwischengeschaltetes Adapterstück 3 miteinander verbunden, wobei das Adapterstück 3 über eine Überwurfmutter 3a mit dem Griffteil 1 verbunden ist und die Nadel 2 über eine Aufnahme 2a am Adapterstück 3 festlegbar ist.

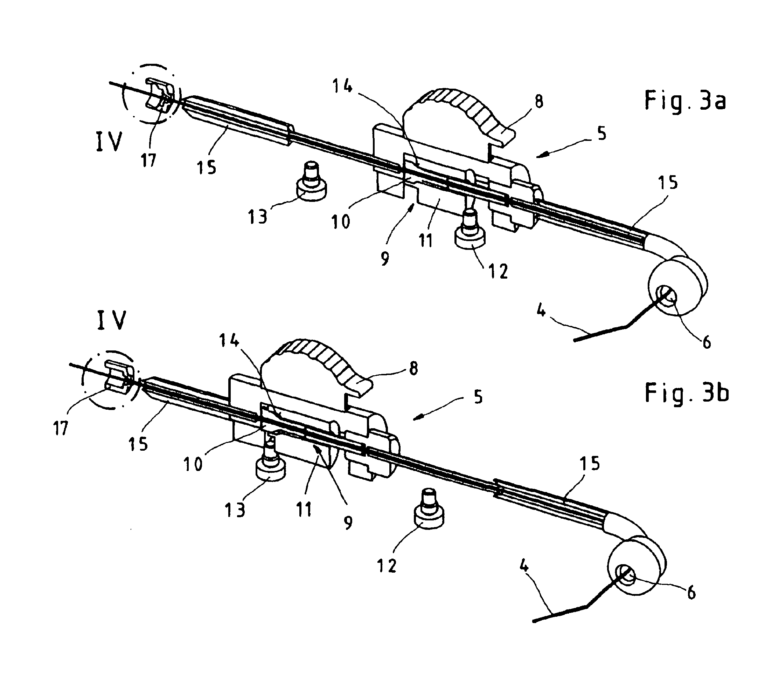

Um Nahtmaterial 4 in die Nadel 2 einzufädeln, ist im Griffteil 1 eine Transportvorrichtung 5 angeordnet, mittels der ausgehend von einer Fadenaufnahme 6 im Griffteil 1 das Nahtmaterial 4 hin zur Nadel 2 transportiert werden kann. Der Aufbau der Transportvorrichtung ist detailliert den Abbildungen Fig. 2a bis 3b zu entnehmen.

Die zwischen zwei Endlagen verschiebbare Transportvorrichtung 5 besteht im wesentlichen aus einem in einem Langloch 7 des Griffteils 1 gelagerten Schieber 8 und einer Einspannvorrichtung 9, die wiederum aus mindestens einem Zangenteil 10 und mindestens einem Klemmelement 11 besteht. Zum Ergreifen des Nahtmaterials 4 besteht das das Nahtmaterial 4 umgebende Zangenteil 10 aus einem radial elastisch verformbaren Material, wie beispielsweise einem Kunststoff-Material. Das radiale Verformen des Zangenteils 10 wird durch das Klemmelement 11 bewirkt, das relativ zu dem Zangenteil 10 verschiebbar derart in dem Schieber 8 der Transportvorrichtung 5 gelagert ist, daß das Klemmelement 11 auf das Zangenteil 10 aufschiebbar und von diesem wieder herunterschiebbar ist.

Die Endlagen, zwischen denen die Transportvorrichtung 5 zum Zuführen des Nahtmaterials 4 hin zur Nadel 2 innerhalb des Griffteils 1 verfahrbar ist, werden durch Anschläge 12 und 13 definiert, die, wie aus Fig. 2a und 2b ersichtlich, im Griffteil 1 angeordnet sind. Wie aus den Abbildungen Fig. 2a bis 3b ersichtlich, bewirkt das Anlaufen der Transportvorrichtung 5 gegen die Anschläge 12 und 13 ein Verschieben des Klemmelements 11 relativ zum Zangenteil 10 und zwar derart, daß beim Anlaufen gegen den hinteren Anschlag 12 das Klemmelement 11 auf das Zangenteil 10 aufgeschoben wird und beim Anlaufen gegen den vorderen Anschlag 13 das Klemmelement 11 wieder vom Zangenteil 10 heruntergeschoben wird.

Aufgrund der radialen Verformbarkeit des Zangenteils 10 bewirkt das Aufschieben des Klemmelements 11, daß das in dem Zangenteil 10 geführte Nahtmaterial 4 von dem Zangenteil 10 so lange klemmend ergriffen wird, bis an der anderen Endlage der Transportvorrichtung 5 diese Klemmwirkung wieder aufgehoben wird, wenn der Anschlag 13 das Klemmelement 11 wieder vom Zangenteil 10 herunter schiebt.

Um das Aufschieben des Klemmteils 11 auf das Zangenteil 10 zu erleichtern, sind bei dem dargestellten Ausführungsbeispiel Anlaufschrägen 14 am Zangenteil 10 ausgebildet. Selbstverständlich ist es auch möglich, Anlaufschrägen14 nur am Klemmelement 11 oder sowohl am Zangenteil 10, als auch am Klemmelement 11 auszubilden.

Das Einfädeln des Nahtmaterials 4 in die Nadel 2 geschieht bei dem dargestellten Naht-Set wie folgt:

Das jeweilige Nahtmaterial 4 wird dem Griffteil 1 über die als radiale Bohrung ausgebildete Fadenaufnahme 6 zugeführt. Zum Stabilisieren und Führen des Nahtmaterials sind innerhalb des Griffteils 1 und des Adapterstücks 3 zumindest abschnittsweise Hohlrohre 15 angeordnet, die das Nahtmaterial führend aufnehmen.

Zu Beginn des Einfädelns muß sich der Schieber 8 und somit die Transportvorrichtung 5 in der in Fig. 1, 2a und 3a dargestellten hinteren Endlage befinden. Das Nahtmaterial 4 wird nun von Hand über die Fadenaufnahme 6 soweit in das Griffteil 1 eingeschoben, bis ein Widerstand spürbar ist. Dann wird die Transportvorrichtung 5 über den Schieber 8 bis in die vordere Endlage am Anschlag 13 verschoben. In dieser Endlage öffnet sich das Zangenteil 10 der Einspannvorrichtung 9 wieder, so daß das Nahtmaterial 4 von Hand weiter in das Griffteil 1 eingeschoben werden kann.

Das solchermaßen manuell in das Griffteil 1 eingeschobene Nahtmaterial 4 tritt nun in das Adapterstück 3 ein. Wie aus Fig. 1 sowie Fig. 2a und 2b ersichtlich, ist in dem Adapterstück 3 wenigstens eine Öffnung 16 ausgebildet, die den Blick auf das eingeführte Nahtmaterial 4 freigibt. Sobald das Nahtmaterial 4 durch die Öffnung 16 erkennbar ist, kann das manuelle Einführen des Nahtmaterials 4 gestoppt werden. Der weitere Transport des Nahtmaterials 4 erfolgt jetzt ausschließlich über die Transportvorrichtung 5. Aus der vorderen Endlage wird die Transportvorrichtung 5 mit geöffnetem Zangenteil 10 der Einspannvorrichtung 9 wieder zur hinteren Endlage verschoben, bis der Anschlag 12 das Klemmelement 11 wieder auf das Zangenteil 10 aufschiebt und so das Nahtmaterial 4 in der Einspannvorrichtung 9 fixiert.

Beim neuerlichen Verschieben der Transportvorrichtung 5 zur vorderen Endlage wird das Nahtmaterial 4 klemmend mitgenommen und weiter in Richtung der Nadel 2 transportiert. Beim Anlaufen gegen den vorderen Anschlag 13 wird das Klemmelement 11 wieder vom Zangenteil 10 herunter geschoben, so daß die Transportvorrichtung 5 wieder in die hintere Endlage verschoben werden kann, ohne das Nahtmaterial wieder zurückzuziehen. Dieser Transportvorgang wird fortgeführt, bis das Nahtmaterial 4 aus der Spitze der Nadel 2 austritt.

Ein versehentliches Zurückziehen des Nahtmaterials 4 aus der Nadel 2 wird bei der dargestellten Ausführungsform eines Naht-Sets weiterhin dadurch verhindert, daß im Übergangsbereich zwischen dem Adapterstück 3 und der Nadel 2 eine Dichtungskappe 17 angeordnet ist, in der zum Durchführen des Nahtmaterials 4 ein Schlitz 17a ausgebildet ist. Insbesondere im Bereich des Schlitzes 17a besteht diese Dichtungskappe 17 aus einem Gummi- oder Kunststoff-Material. Aufgrund der Reibung zwischen dem Nahtmaterial 4 und den an dem Nahtmaterial 4 anliegenden Seitenwänden des Schlitzes 17a wird ein Zurückziehen des Nahtmaterials 4 aus der Nadel 2 beim Zurückziehen der Transportvorrichtung 5 verhindert.

Ein solchermaßen ausgestaltetes Naht-Set zeichnet sich dadurch aus, daß das Einfädeln des Nahtmaterials 4 nach dem anfänglichen manuellen Einführen vollständig mechanisch ohne manuellen Angriff am Nahtmaterial 4 und darüber hinaus ohne Knicken des Nahtmaterials 4 erfolgt.

### Bezugszeichenliste

- 1: Handgriff
- 2: Nadel
- 2a: Aufnahme
- 3: Adapterstück
- 3a: Überwurfmutter
- 4: Nahtmaterial
- 5: Transportvorrichtung
- 6: Fadenaufnahme
- 7: Langloch
- 8: Schieber
- 9: Einspannvorrichtung
- 10: Zangenteil
- 11: Klemmelement
- 12: Anschlag
- 13: Anschlag
- 14: Anlaufschräge
- 15: Hohlrohr
- 16: Öffnung
- 17: Dichtungskappe
- 17a: Schlitz

## Patentansprüche

1. Naht-Set für medizinische Zwecke, mit einem Griffteil (1), einer an dem Griffteil (1) festlegbaren, zumindest teilweise als Hohlnadel ausgebildeten Nadel (2) sowie einer in dem Griffteil (1) angeordneten, zwischen zwei Endlagen verschiebbaren Transportvorrichtung (5) zum Zuführen von biegeschlaffem Nahtmaterial (4) zur Nadel (2), wobei die Transportvorrichtung (5) eine Einspannvorrichtung (9) aufweist, über die das eingeführte Nahtmaterial (4) in einer Endlage der Transportvorrichtung (5) klemmend ergreifbar und erst in der anderen Endlage der Transportvorrichtung (5) wieder freigebbar ist,
**dadurch gekennzeichnet,**
**daß** die Einspannvorrichtung (9) aus mindestens einem das Nahtmaterial (4) umgebenden, radial elastisch verformbaren Zangenteil (10) und mindestens einem relativ zu dem Zangenteil (10) und auf das Zangenteil (10) aufschiebbaren Klemmelement (11) besteht, wobei das Klemmelement (11) derart relativ zu dem mindestens einen Zangenteil (10) verschiebbar ist, daß beim Anlaufen der Transportvorrichtung (5) gegen die eine Endlage das Klemmelement (11) auf das Zangenteil (10) aufschiebbar ist und beim Anlaufen der Transportvorrichtung (5) gegen die andere Endlage das Klemmelement (11) wieder von dem Zangenteil (10) herunterschiebbar ist.

2. Naht-Set nach Anspruch 1, **dadurch gekennzeichnet, daß** die Einspannvorrichtung (9) innerhalb eines manuell zwischen den beiden Endlagen der Transportvorrichtung (5) verstellbaren Schiebers (8) angeordnet ist.

3. Naht-Set nach Anspruch 1 oder 2, **dadurch gekennzeichnet, daß** im Griffteil (1) angeordnete Anschläge (12, 13) die Endlagen der Transportvorrichtung (5) definieren.

4. Naht-Set nach Anspruch 3, **dadurch gekennzeichnet, daß** die Anschläge (12, 13) am verschiebbaren Klemmelement (11) angreifen.

5. Naht-Set nach einem der Ansprüche 1 bis 4, **dadurch gekennzeichnet, daß** am Zangenteil (10) und/oder am Klemmelement (11) Anlaufschrägen (14) ausgebildet sind.

6. Naht-Set nach Anspruch 5, **dadurch gekennzeichnet, daß** die Nadel (2) über ein Adapterstück (3) am Griffteil (1) festlegbar ist.

7. Naht-Set nach Anspruch 6, **dadurch gekennzeichnet, daß** in dem Adapterstück (3) wenigstens eine einen Blick auf das Nahtmaterial (4): freigebende Öffnung (16) ausgebildet ist.

8. Naht-Set nach Anspruch 6 oder 7, **dadurch gekennzeichnet, daß** im Übergangsbereich vom Adapterstück (3) zur Nadel (2) eine mit einem Schlitz (17a) zum Durchführen des Nahtmaterials (4) versehene Dichtungskappe (17) angeordnet ist.

9. Naht-Set nach Anspruch 8, **dadurch gekennzeichnet, daß** die Dichtungskappe (17) zumindest im Bereich des Schlitzes (17a) aus einem elastischen Material, insbesondere einem Gummi- oder Kunststoff-Material, besteht.

10. Naht-Set nach Anspruch 6, **dadurch gekennzeichnet, daß** im Griffteil (1) und im Adapterstück (3) zumindest abschnittsweise Hohlrohre (15) zum Führen des Nahtmaterials (4) angeordnet sind.

11. Naht-Set nach einem der Ansprüche 1 bis 10, **dadurch gekennzeichnet, daß** das Nahtmaterial (4) über eine als radiale Bohrung ausgebildete Fadenaufnahme (6) in das Griffteil (1) und die Transportvorrichtung (5) einführbar ist.

## Claims

1. Suturing set for medical use, with a handle (1), with a needle (2) which can be secured on the handle (1) and is designed at least partially as a hollow needle, and with a transport device (5) which is arranged in the handle (1) and can be displaced between two end positions in order to deliver flexurally slack suture material (4) to the needle (2), the transport device (5) having a gripping device (9) by means of which the inserted suture material (4) can be grasped with a clamping action in one end position of the transport device (5) and can be released again only in the other end position of the transport device (5),
**characterized in that** the gripping device (9) is composed of at least one pincer part (10), which surrounds the suture material (4) and is radially elastically deformable, and of at least one clamping element (11), which can be pushed relative to the pincer part (10) and onto said pincer part (10), the clamping element (11) being displaceable relative to the at least one pincer part (10) in such a way that, when the transport device (5) runs up against the one end position, the clamping element (11) can be pushed onto the pincer part (10) and, when the transport device (5) runs up against the other end position, the clamping element (11) can be pushed down again from the pincer part (10).

2. Suturing set according to Claim 1, **characterized in that** the gripping device (9) is arranged inside a slide (8) that can be shifted manually between the two end positions of the transport device (5).

3. Suturing set according to Claim 1 or 2, **characterized in that** limit stops (12, 13) arranged in the handle (1) define the end positions of the transport device (5).

4. Suturing set according to Claim 3, **characterized in that** the limit stops (12, 13) engage the displaceable clamping element (11).

5. Suturing set according to one of Claims 1 to 4, **characterized in that** run-up bevels (14) are formed on the pincer part (10) and/or on the clamping element (11).

6. Suturing set according to Claim 5, **characterized in that** the needle (2) can be secured on the handle (1) via an adapter piece (3).

7. Suturing set according to Claim 6, **characterized in that** the adapter piece (3) has at least one opening (16) providing a view of the suture material (4).

8. Suturing set according to Claim 6 or 7, **characterized in that** a sealing cap (17), provided with a slit (17a) for passage of the suture material (4), is arranged in the transition area from the adapter piece (3) to the needle (2).

9. Suturing set according to Claim 8, **characterized in that** the sealing cap (17), at least in the area of the slit (17a), is made of an elastic material, in particular a rubber or plastic material.

10. Suturing set according to Claim 6, **characterized in that** hollow tubes (15) for guiding the suture material (4) are arranged in at least some areas in the handle (1) and in the adapter piece (3).

11. Suturing set according to one of Claims 1 to 10, **characterized in that** the suture material (4) can be inserted into the handle (1) and the transport device (5) via a thread receiver (6) designed as a radial bore.

## Revendications

1. Ensemble de suture destiné à des applications médicales, comportant un manche (1), une aiguille (2), apte à être fixée au manche (1) et réalisée au moins partiellement sous forme d'aiguille creuse, ainsi qu'un dispositif de transport (5), agencé dans le manche (1), pouvant coulisser entre deux positions finales et destiné à acheminer le matériau de suture (4) lâche et flexible vers l'aiguille (2), le dispositif de transport (5) comportant un dispositif de blocage (9), par lequel, dans une position finale du dispositif de transport (5), le matériau de suture (4) introduit peut être saisi en étant bloqué et, uniquement dans l'autre position finale du dispositif de transport (5), peut à nouveau être libéré,
**caractérisé en ce que** le dispositif de blocage (9) est formé par au moins une pince (10), enserrant le matériau de suture (4) et pouvant être déformée élastiquement dans le sens radial, et au moins un élément de serrage (11), pouvant être emmanché sur la pince (10) et pouvant coulisser par rapport à la pince (10), l'élément de serrage (11) étant apte à coulisser par rapport à ladite au moins une pince (10) de telle sorte que, lorsque le dispositif de transport (5) vient buter contre l'une des positions finales, l'élément de serrage (11) peut être emmanché sur la pince (10) et, lorsque le dispositif de transport (5) vient buter contre l'autre position finale, l'élément de serrage (11) peut être retiré de la pince (10).

2. Ensemble de suture selon la revendication 1, **caractérisé en ce que** le dispositif de blocage (9) est agencé à l'intérieur d'un curseur (8) pouvant être déplacé manuellement entre les deux positions finales du dispositif de transport (5).

3. Ensemble de suture selon la revendication 1 ou 2, **caractérisé en ce que** des butées (12, 13), agencées dans le manche (1), définissent les positions finales du dispositif de transport (5).

4. Ensemble de suture selon la revendication 3, **caractérisé en ce que** les butées (12, 13) entrent en contact avec l'élément de serrage (11) mobile.

5. Ensemble de suture selon l'une quelconque des revendications 1 à 4, **caractérisé en ce que** des plans inclinés (14) sont réalisés sur la pince (10) et/ou l'élément de serrage (11).

6. Ensemble de suture selon la revendication 5, **caractérisé en ce que** l'aiguille (2) est apte à être fixée sur le manche (1) par l'intermédiaire d'un adaptateur (3).

7. Ensemble de suture selon la revendication 6, **caractérisé en ce qu'**au moins une fenêtre (16), permettant de voir le matériau de suture (4), est réalisée dans l'adaptateur (3).

8. Ensemble de suture selon la revendication 6 ou 7, **caractérisé en ce que** dans la zone de transition entre l'adaptateur (3) et l'aiguille (2) est disposé un capuchon d'étanchéité (17), muni d'une fente (17a) pour le passage du matériau de suture (4).

9. Ensemble de suture selon la revendication 8, **caractérisé en ce que** le capuchon d'étanchéité (17) est réalisé, au moins dans la zone de la fente (17a), dans un matériau élastique, en particulier un matériau à base de caoutchouc ou une matière plastique.

10. Ensemble de suture selon la revendication 6, **caractérisé en ce que** des tubes creux (15) pour le guidage du matériau de suture (4) sont disposés au moins par zones dans le manche (1) et dans l'adaptateur (3).

11. Ensemble de suture selon l'une quelconque des revendications 1 à 10, **caractérisé en ce que** le matériau de suture (4) peut être introduit dans le manche (1) et le dispositif de transport (5) en passant par un élément de réception du fil (6), réalisé sous forme de forure radiale.
